(19) Europäisches Patentamt
European Patent Office
Office européen des brevets

(11) EP 0 940 404 A1

(12) DEMANDE DE BREVET EUROPEEN

(43) Date de publication:
**08.09.1999 Bulletin 1999/36**

(21) Numéro de dépôt: **98403312.6**

(22) Date de dépôt: **28.12.1998**

(51) Int. Cl.$^6$: **C07F 7/08**, C07F 7/18, C07F 7/21, C08G 77/388, A61K 7/06

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **02.03.1998 FR 9802486**

(71) Demandeur: **L'OREAL**
**75008 Paris (FR)**

(72) Inventeur: **Richard, Hervé**
**93420 Villepinte (FR)**

(74) Mandataire: **Miszputen, Laurent**
**L'Oreal,**
**DPI,**
**6 rue Sincholle**
**92585 Clichy Cédex (FR)**

Remarques:
Une requête en rectification portant sur la numérotation des revendications a été présentée conformément à la règle 88 CBE. Il est statué sur cette requête au cours de la procédure engagée devant la division d'examen (Directives relatives à l'examen pratiqué à l'OEB, A-V, 3.).

# (54) Utilisation d'un organosiloxane et/ou d'un organosilane antioxydant pour la photoprotection de la couleur des fibres kérantiniques colorées naturellement ou artificiellement

(57) La présente demande concerne l'utilisation d'un organosiloxane et/ou d'un organosilane porteur d'au moins un groupe antioxydant dans et pour la préparation d'une composition cosmétique ou dermatologique comme agent de protection de la couleur des fibres kératiniques colorées naturellement ou teintes artificiellement contre les effets des radiations UV en particulier du rayonnement solaire.

EP 0 940 404 A1

## Description

La présente demande concerne l'utilisation d'un organosiloxane et'ou d'un organosilane porteur d'au moins un groupe antioxydant dans et pour la préparation d'une composition cosmétique ou dermatologique comme agent de protection de la couleur des fibres kératiniques colorées naturellement ou teintes artificiellement contre les effets des radiations UV en particulier du rayonnement solaire.

On sait depuis longtemps que notamment la lumière et les agents de lavage ont tendance à agresser la couleur naturelle des cheveux ou la teinture artificielle des cheveux. La couleur des cheveux obtenue après un traitement de teinture s'affaiblit peu à peu ou vire vers des nuances peu esthétiques ou indésirables.

Il est donc apparu nécessaire de protéger la couleur naturelle des cheveux ainsi que la couleur artificielle des cheveux après un traitement de teinture et de réduire sensiblement les dégradations liées à l'action des agents extérieurs, notamment les radiations UV et plus particulièrement le rayonnement solaire.

La Demanderesse a découvert ce qui fait l'objet de l'invention, que des silanes et/ou des silicones portant des groupements antioxydants, préservaient, de façon surprenante, la couleur des fibres kératiniques colorées naturellement ou teintes artificiellement ainsi que leurs teintes obtenues artificiellement, en réduisant sensiblement les dégradations liées à l'action des rayonnements UV, en particulier l'action du rayonnement solaire.

L'invention a donc pour objet l'utilisation d'au moins un organosiloxane et/ou d'au moins un organosilane porteur d'au moins un groupe antioxydant dans et pour la préparation d'une composition cosmétique ou dermatologique comme agent de protection de la couleur des fibres kératiniques colorées naturellement ou teintes artificiellement contre les effets néfastes des rayonnements UV, en particulier du rayonnement solaire.

D'autres objets apparaîtront à la lecture de la description et des exemples qui suivent.

Les organosiloxanes et les organosilanes portant des groupements antioxydants, utilisés selon l'invention, comportent dans leur molécule au moins une unité de formule :

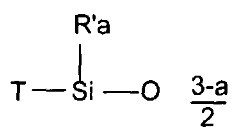

(I)

dans laquelle :

- $R'_a$, identiques ou différents, désignent un groupe hydrocarboné saturé ou insaturé en $C_1$-$C_{30}$, un groupe hydrocarboné halogéné en $C_1$-$C_8$, un groupe hydrocarboné aromatique, un groupe alkoxy en $C_1$-$C_{10}$,
- a = 1, 2 ou 3 ;
- T = -E-U où E représente un radical divalent hydrocarboné aliphatique, saturé ou insaturé, linéaire ou ramifié ayant au moins 2 atomes de carbone et renfermant éventuellement un ou plusieurs atomes d'oxygène ou bien un groupement carboxylique, amide ou urée ou bien un groupe aromatique ou bien un radical hydroxyle et U représente le reste d'une molécule antioxydante.

En plus des unités de formule (I), l'organosiloxane peut comporter des unités de formule (II) et/des unités de formule (III) :

$$R'b - Si - O_{\frac{4-b}{2}} \qquad (II)$$

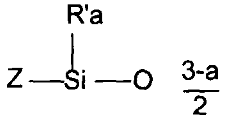

(III)

dans lesquelles :

- R' et a ont les même significations que dans la formule (I);
- b = 1, 2 ou 3 ;
- Z = -O-U, U ayant la même signification que dans la formule (I).

A titre de groupe hydrocarboné, on peut citer les radicaux alkyle en $C_1$-$C_{30}$, alcényle en $C_2$-$C_{30}$, cycloalkyle, aromatique comme phényle ou toluyle.

A titre de groupe hydrocarboné halogéné, on peut citer le radical 3,3,3-trifluoropropyle.

Dans les organosiloxanes conformes à l'invention, constitués d'unités de formule (I) et éventuellement d'unités de formule (II) et/ou de formule (III), de façon préférentielle, au moins 40% en nombre des radicaux R' sont des radicaux méthyle. Le nombre total des unités (I), (II) et (III) est de préférence inférieur ou égal a 250 et varie en particulier de 2 à 50.

U représente de préférence un reste :

- un radical phénol mono ou di-ortho substitué et dont le noyau benzénique peut être substitué par un ou plusieurs radicaux alkyle, halogène, hydroxy, alkoxy; alkyle carbonyle;
- un dérivé 1,1,6,6-tetrasubstitué de piperidine (HALS);
- un groupe oxamide ou oxanilide.

Les organosiloxanes porteurs d'au moins un groupe antioxydant selon l'invention sont choisis en particulier parmi ceux à fonction ter-butyl phénol et/ou dérivé 1,1,6,6-tetrasubstitué de piperidine et/ou dérivé d'oxamide et/ou dérivé d'oxanilide qui répondent aux formules suivantes:

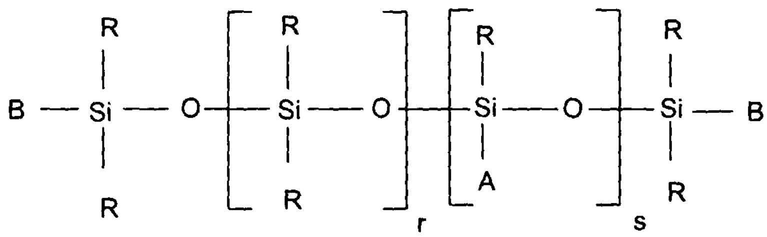

(1)

ou

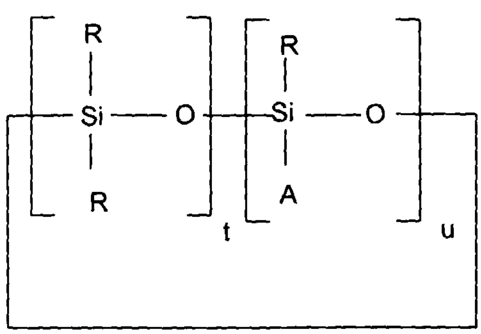

(2)

et les organosilanes porteurs d'au moins un groupe antioxydant de l'invention sont choisis en particulier parmi ceux à fonction ter-butyl phénol et/ou dérivé 1,1,6,6-tetrasubstitué de piperidine et/ou dérivé d'oxamide et/ou dérivé d'oxanilide qui répond à la formule suivante :

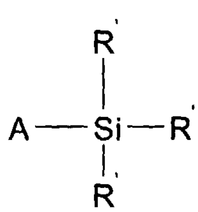

(3)

formules (1), (2) et (3) dans lesquelles :

- R, identiques ou différents, sont choisis parmi les radicaux alkyles en $C_1$-$C_{10}$, phényle et trifluoro-3,3,3 propyle, au

moins 80% en nombre des radicaux R étant méthyle,

- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- R', identiques ou différents sont choisis parmi les radicaux alkyles en $C_1$-$C_{10}$, linéaires ou ramifiés ou les radicaux alkoxy en $C_1$-$C_8$,
- et le symbole A désigne un radical monovalent lié directement à un atome de silicium, et qui répond aux formules (4a), (4b) et (4c) suivantes :

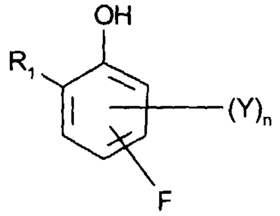

(4a)

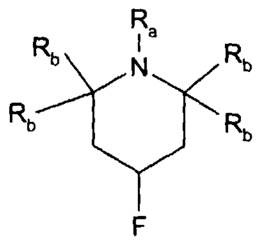

(4b)

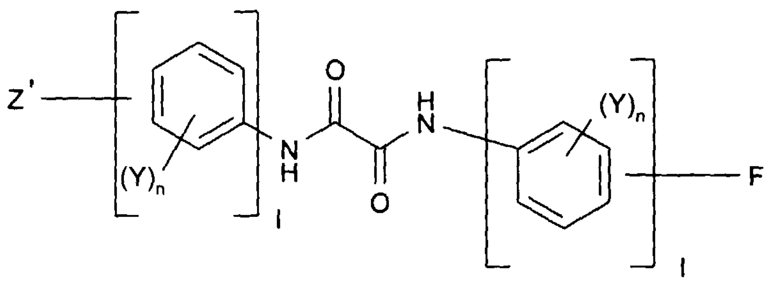

(4c)

dans lesquelles : F est le biradical de formule (5) suivante :

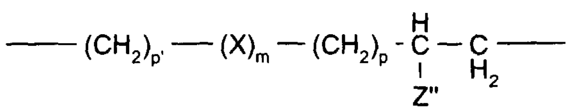

(5)

- $R_1$ est un radical alkyle en $C_3$-$C_{12}$ ramifié ou un radical alkoxy en $C_1$- $C_4$,
- Y, identiques ou différents, sont choisis parmi les radicaux alkyles en $C_1$-$C_{12}$, linéaires ou ramifiés, les radicaux cycloalkyles en $C_5$-$C_7$, le radical hydroxyle, les radicaux alkyle alkoxy, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- n est un nombre entier compris entre 0 et 2 inclusivement,
- X représente O ou $NR_c$ ou O(C=O) ou $NR_c$(C=O) ou $NR_c$(C=O)O avec $R_c$ qui représente l'hydrogène ou un radical alkyle en $C_1$-$C_4$,
- Z" représente l'hydrogène, un radical alkyle en $C_1$-$C_4$ ou un radical hydroxyle,
- m est 0 ou 1,
- p et p' représentent un nombre entier compris entre 0 et 10, inclusivement.
- $R_a$ représente l'hydrogène, l'oxygène (radical nitroxyde), un radical alkyle en $C_1$-$C_8$, un radical hydroxyle, un radical

hydroxyalkyle en $C_2$-$C_4$, un radical alkylcarbonyle, phényle, benzyle ou un radical alkoxy en $C_1$-$C_6$ linéaire ou cyclique

- $R_b$ représente un radical alkyle en $C_1$-$C_8$
- I est 0 ou 1,
- Z' représente le radical divalent F, l'hydrogène ou une chaîne hydrocarbonée, linéaire ou ramifié, saturée ou insaturée comportant jusqu'à 20 atomes de carbone consécutifs, pouvant comporter un ou plusieurs groupements hydroxyle et pouvant être interrompue par un ou plusieurs atomes d'oxygène ou d'azote et/ou plusieurs cycles aromatiques.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (1) ou (2) ci-dessus, on préfère mettre en oeuvre ceux répondant à la formule (1), c'est-à-dire des diorganosiloxanes à chaîne courte linéaire, ou des organosilanes.

Parmi les composés de formules (1) ci-dessus, on préfère mettre en oeuvre ceux pour lesquels les radicaux B sont tous les deux des radicaux R.

Parmi les diorganosiloxanes linéaires ou les organosilanes rentrant dans le cadre de la présente invention, on préfère plus particulièrement les dérivés statistiques ou bien définis à blocs présentant au moins l'une, et encore plus préférentiellement l'ensemble, des caractéristiques suivantes :

- B est un radical R,
- R est alkyle et encore plus préférentiellement est méthyle,
- r est compris entre 0 et 15 inclusivement; s est compris entre 1 et 10 inclusivement,
- n est non nul, et de préférence égal à 1, et Y est alors choisi parmi méthyle, ter.-butyle ou alcoxy en $C_1$-$C_4$,
- Z" est hydrogène ou méthyle,
- m=0, ou [m=1 et X=O]
- p est égal à 1
- R' est méthyle et/ou éthyle.

Une famille de composés convenant particulièrement à l'invention est celle définie par la formule générale (6) suivante :

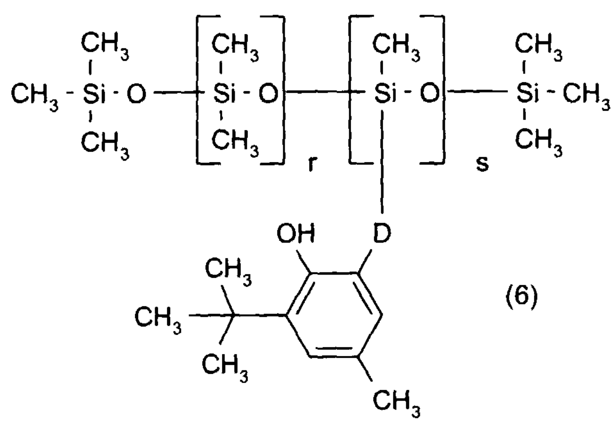

(6)

avec

$$0 \leq r \leq 6,$$

$$1 \leq s \leq 6,$$

et où D représente le radical divalent :

$$-CH_2-CH_2-CH_2-$$

Parmi les composés de formule (6), on préfère plus particulièrement la silicone antioxydante répondant à la formule suivante :

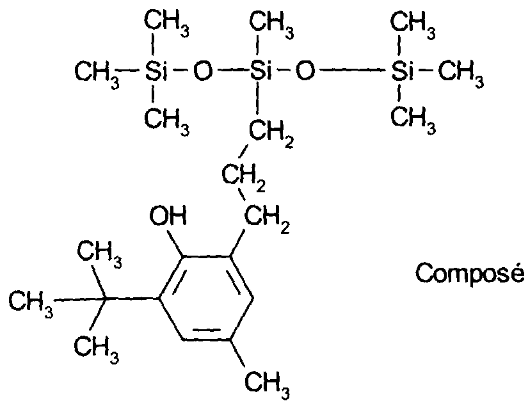

Composé A

Une famille de composés convenant particulièrement à l'invention est celle définie par la formule générale (7) suivante :

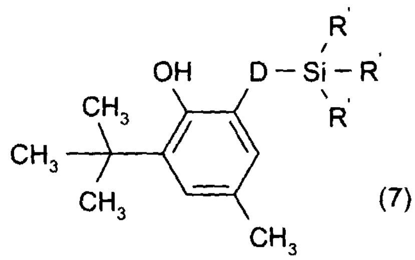

(7)

ou R' a la même définition qu'au dessus
et où D représente le radical divalent :

$$—CH_2—CH_2—CH_2—$$

Parmi les composés de formule (7), on préfère plus particulièrement la silicone antioxydante (appelé composé B dans la suite du texte) répondant à la formule suivante :

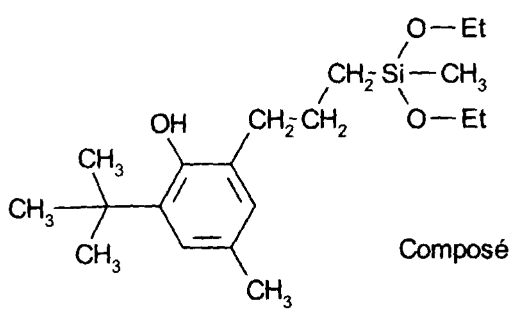

Composé B

Une autre famille de silicones antioxydantes préférées comprend les silicones piperidinyle tetraalkylées répondant à la formule générale suivante (8) :

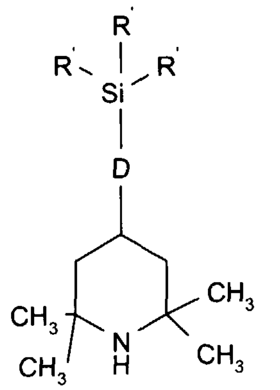

(8)

ou $R'$ a la même définition qu'au dessus :
et où D représente le radical divalent :

$$—O—CH_2—CH_2—CH_2—$$

Parmi les composés de formule (8), on préfère plus particulièrement la silicone antioxydante (appelé composé C dans la suite du texte) répondant à la formule suivante :

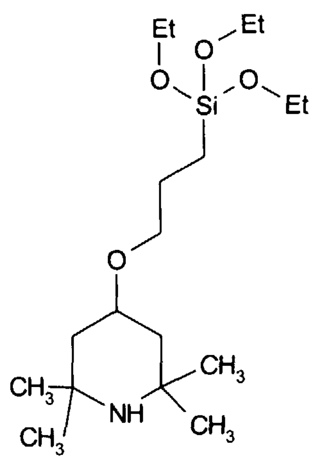

Composé C

Une autre famille de silicones antioxydantes préférées comprend les silicones d'oxamides répondant à la formule générale suivante (9) :

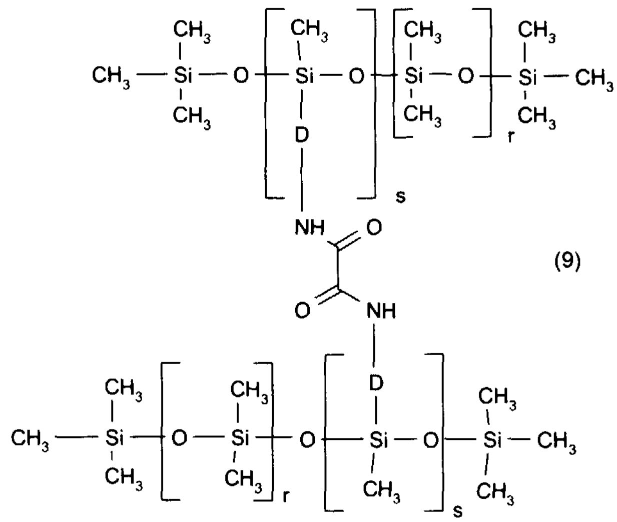

(9)

avec

$$0 \leq r \leq 6,$$

$$1 \leq s \leq 6,$$

et où D représente le radical divalent :

$$—CH_2—CH_2—CH_2—$$

Parmi les composés de formule (9), on préfère plus particulièrement la silicone antioxydante que l'on appellera composé E répondant à la formule suivante :

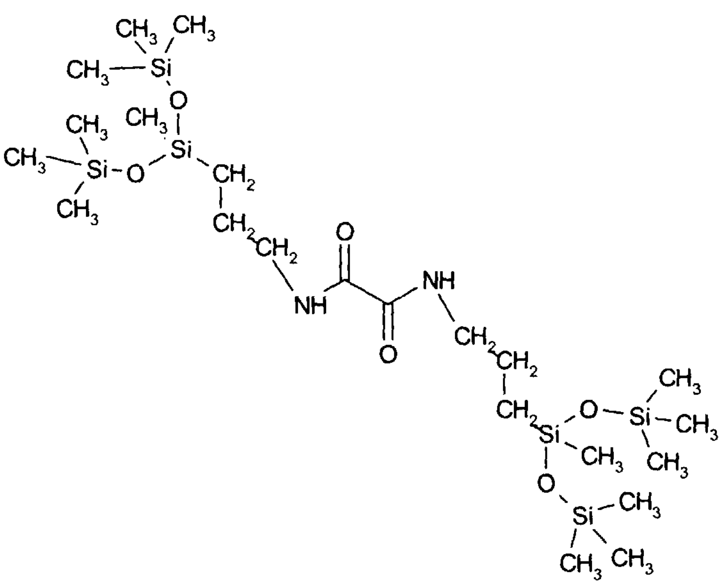

composé E

Les procédés de synthèse de ces silicones ou organosilanes antioxydants sont décrites dans les demandes de brevets référencés ci-dessous.

Les organosiloxanes porteurs de groupements phénoliques ortho substitués sont décrits notamment dans les

demandes de brevet européen n° 0370868, 0776946; les alkoxysilanes porteurs de groupements phénoliques ortho substitués sont décrits notamment dans les brevets américains USP n° 3328450, 3579467, 4430235, dans la demande de brevet de Grande Bretagne n° 2030581 et les demandes de brevets japonais n° 84 124923 et n° 86 100587. Les organosiloxanes porteurs de groupements phénoliques di-ortho substitués sont décrits notamment dans les demandes de brevet européen n° 0162523, 0390153, 0393239, dans les brevets américains USP 3579467, 4879378, 4960810; les organosilanes porteurs de groupements phénoliques di-ortho substitués sont décrits notamment dans les demandes de brevet américain USP n° 5608095. Toutefois parmi ces composés, un certain nombre sont nouveaux et ceci constitue un autre objet de la présente invention. Il s'agit des composés silanes répondant à l'une des formules (10a) et (10b) suivantes :

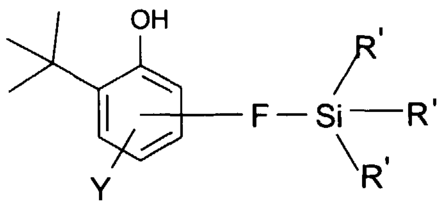

(10a)

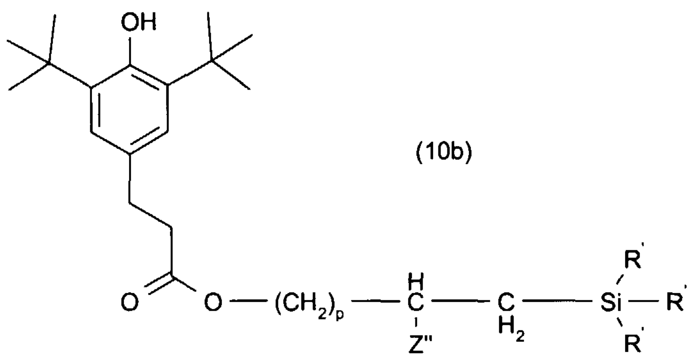

(10b)

dans lesquelles F, p, Z'', Y et R' ont la même signification qu'au dessus; sous réserve que R' soit différent d'un radical alcoxy.

Les organopolysiloxanes porteurs de groupements piperidinyles (HALS) sont décrits notamment dans les demandes de brevet européen n° 0307054, 0343717, 0358190, 0388321, 0659930, 0665233, 0664315; les organopolysiloxanes porteurs de groupements 1-hydrocarbyloxy piperidinyles sont décrits notamment dans la demande de brevet européen n° 0665233; les alkoxysilanes porteurs de groupements piperidinyles (HALS) sont décrits notamment dans les demandes de brevet européen n°0162524, 0491659 et 0573113. Toutefois parmi ces composés, un certain nombre sont nouveaux et ceci constitue un autre objet de la présente invention. Il s'agit des dérivés organosilanes appartenant à la formule (11) suivante :

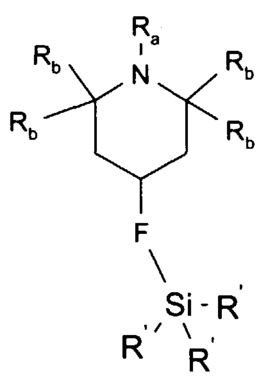

(11)

dans laquelle $R_a$, $R_b$, F et R' ont la même signification qu'au dessus; sous réserve que R' soit différent d'un radical alcoxy.

Les organopolysiloxanes porteurs de groupements oxanilides sont décrits notamment dans la demande de brevet européen n° 0555893.

Les organopolysiloxanes porteurs de groupements oxamides sont décrits notamment dans la demande de brevet européen n° 0712856.

Toutefois parmi ces composés, un certain nombre sont nouveaux et ceci constitue un autre objet de la présente invention. Il s'agit des dérivés organosiloxanes de formule (1) ou (2) telles que définies précédemment dans lesquelles A répond à la formule :

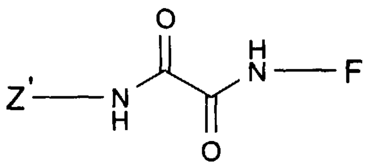

où F et Z' ont la même signification qu'au dessus.

Un autre famille de nouveaux organosilanes à fonction antioxydante conformes à l'invention répond à la formule (12) suivante :

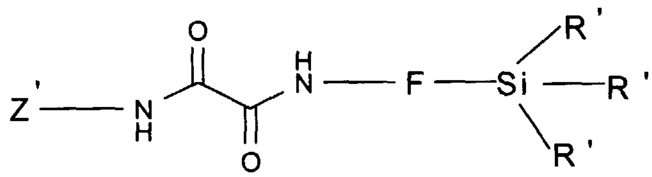

(12)

où F, Z' et R' ont la même signification qu'au dessus; sous réserve que R' soit différent d'un radical alcoxy.

Un autre objet de l'invention porte sur l'utilisation des nouveaux organosiloxanes et/ou organosilanes antioxydants tels que décrits ci-dessus pour la préparation d'une composition cosmétique ou dermatologique comme agent de protection de la couleur des fibres kératiniques colorées naturellement ou teintes artificiellement contre les effets néfastes des rayonnements UV, en particulier du rayonnement solaire.

Un autre objet de l'invention comprend les compositions cosmétiques ou dermatologiques photoprotectrices de la couleur naturelle ou artificielle des fibres kératiniques telles que cheveux contenant dans un support cosmétique au moins l'un des nouveaux organosiloxanes et/ou organosilanes antioxydants tels que décrits ci-dessus.

Une forme particulière de l'invention consiste à associer à l'une des silicones ou l'un des organosilanes antioxydants tels que définis ci-dessus au moins un organosiloxane porteur d'au moins un groupe absorbant les ultraviolets.

Un autre objet de la présente invention concerne donc une composition photo protectrice de la couleur naturelle ou artificielle des fibres kératiniques, en particulier des cheveux humains, comprenant dans un support cosmétiquement acceptable au moins une silicone antioxydante telle que définie ci-dessus et au un organosiloxane porteur d'au moins un groupe absorbant les ultraviolets.

Les organosiloxanes porteurs de groupes absorbant les ultraviolets peuvent être ceux décrits notamment dans les demandes de brevet européen EP0335777, EP0305059, EP0392882, EP0392883, EP0388218, EP0350314, EP0383655, EP038933, EP0709080, EP0538431, dans la demande de brevet internationale WO 92/20690, dans les demandes de brevet français FR 2550787 et FR 2657351 et dans les brevets américains USP 4696969, USP 4554369, USP 4562278, USP 3513184,USP 4859759.

On utilisera plus particulièrement la silicone filtre benzotriazole (appelé composé G dans la suite du texte) répondant à la formule suivante :

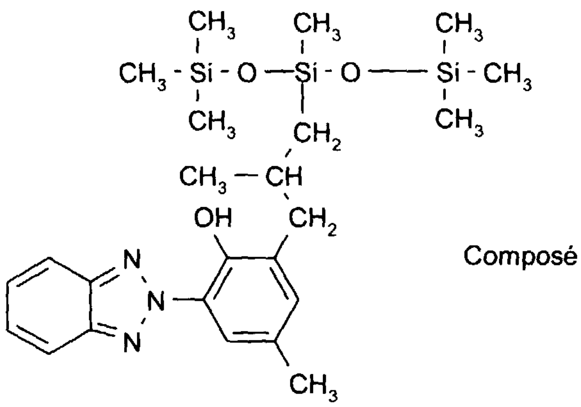

Composé G

Les organosiloxanes et/ou les organosilanes antioxydants de l'invention sont utilisés de préférence dans des quantités au moins égales à 0,01% en poids et généralement allant de 0,1 à 20% en poids et plus particulièrement de 0,1 à 10% en poids par rapport au poids de la composition destinée à être appliquée sur les fibres kératiniques colorées naturellement ou teintes artificiellement.

Les organosiloxanes filtres de l'invention sont utilisés de préférence dans des quantités au moins égales à 0,01% en poids et généralement allant de 0,1 à 20% en poids et plus particulièrement de 0,1 à 10% en poids par rapport au poids de la composition destinée à être appliquée sur les fibres kératiniques colorées naturellement ou teintes artificiellement.

Les compositions destinées à être appliquées sur les fibres kératiniques colorées, conformément à l'invention peuvent se présenter sous forme de lotion huileuse, alcoolique ou hydroalcoolique, sous forme d'émulsion, sous forme de dispersion aqueuse ou hydroalcoolique.

Lorsque les compositions constituent des lotions huileuses, elles contiennent outre l'organosiloxane et/ou l'organosilane porteur d'un groupe antioxydant, des huiles minérales, végétales, animales ou synthétiques et plus particulièrement des isoparaffines ou des huiles de silicone à structure linéaire ou cyclique comme les polyalkyl-siloxanes, les polyarylsiloxanes, les polyalkylarylsiloxanes ou les polyorganosiloxanes modifiés par des groupements organofonctionnels non-chromophores.

Les compositions huileuses peuvent également contenir des cires, des résines ou des gommes de silicone conjointement aux huiles définies ci-dessus.

Les lotions alcooliques contiennent, outre l'organosiloxane et/ou l'organosilane porteur d'un groupe antioxydant, par exemple un alcool inférieur comportant 1 à 4 atomes de carbone et de préférence l'éthanol ou l'isopropanol ou bien d'autres alcools tels que les alkylèneglycols ou les éthers de glycols.

Lorsque les compositions de l'invention sont des émulsions, la phase grasse des émulsions est constituée, soit uniquement de l'organosiloxane et/ou de l'organosilane antioxydant, soit par un mélange de celui-ci avec d'autres huiles ou cires telles que définies précédemment. L'autre phase des émulsions est constituée par un milieu aqueux.

Les émulsions non-ioniques de l'invention contiennent un émulsionnant non-ionique choisi par exemple parmi les alcools gras polyoxyéthylénés, les acides gras polyoxyéthylénés, les esters de sorbitan éventuellement polyoxyéthylénés, les alkylphénols polyoxyéthylénés ou polyglycérolés, les amides gras polyoxyéthylénés ou polyglycérolés, les alcools gras et alpha-diols polyglycérolés ; le nombre de moles d'oxyde d'éthylène variant de préférence de 2 à 50 et le nombre de groupements polyglycérolés variant de préférence de 2 à 30.

Les émulsions cationiques de l'invention contiennent un émulsionnant cationique choisi par exemple parmi les halogénures d'ammonium quaternaire tels que de dialkyl ($C_{10}$-$C_{30}$) diméthylammonium, d'alkyl ($C_{10}$-$C_{30}$) triméthylammonium, ou d'alkyl($C_{10}$-$C_{30}$) benzyl diméthylammoium et les sels d 'ammoniums quaternaires polyoxyéthylénés comportant de 2 à 30 moles d'oxyde d'éthylène. On utilise plus particulièrement le chlorure de distéaryl diméthylammonium et le chlorure de béhényl triméthylammonium.

Lorsque les compositions de l'invention sont des dispersions, elles contiennent en général, de l'eau et éventuellement un alcool tel que ceux indiqués précédemment, un agent de dispersion ou un agent de mise en suspension de la silicone filtre dans l'eau. On peut citer, par exemple, comme agent de dispersion, les copolymères acrylate d'ammonium/acrylamide et les polymères d'acide acrylique réticulé.

Les compositions conformes à l'inventions peuvent également tout autre additif habituellement appliqué sur les fibres kératiniques et en particulier les cheveux tels que par exemple des colorants, des tensioactifs, des polymères, des épaississants, des agents de conditionnement, des parfums, des conservateurs, des adoucissants ainsi que

d'autres agents filtrants.

Les compositions conformes à l'invention peuvent également se présenter sous forme de spray ou être pressurisées dans des dispositifs aérosols.

Les compositions de l'invention destinées à être appliquées sur des fibres kératiniques colorées artificiellement et notamment des cheveux humains, sont des produits rincés appliqués après teinture tels que des shampooings, des après-shampooings ou bien des produits non-rincés appliqués après teinture tels que des lotions, des gels, des mousses de coiffage, des laques aérosols ou des spray.

Les fibres kératiniques teintes artificiellement et en particulier les cheveux, sont colorés en mettant en oeuvre des colorants classiquement utilisés en capillaire tels que des précurseurs de colorant d'oxydation selon un processus de coloration mettant en oeuvre un agent oxydant tel que l'eau oxygénée ou l'air ou bien par coloration directe. On peut aussi envisager la teinture des fibres kératiniques en présence de l'organosiloxane et/ou de l'organosilane antioxydant.

On peut également envisager d'appliquer une composition selon l'invention à base de l'organosiloxane et/ou l'organosilane antioxydant avant la teinture des fibres kératiniques.

La présente invention a pour objet également un procédé de traitement cosmétique destiné à protéger la coloration des cheveux teints contre les effets néfastes des rayonnements UV et en particulier du rayonnement solaire qui consiste à appliquer avant ou après teinture des cheveux une quantité efficace d'une composition cosmétique ou dermatologique contenant au moins un composé organosiloxane et/ou un composé organosilane tel que défini ci-dessus.

Les exemples qui suivent servent à illustrer l'invention sans toutefois présenter un caractère limitatif.

## EXEMPLES DE PREPARATION

Les exemples ci-après illustrent l'invention sans en imiter la portée.

- EXEMPLE 1 : Appelé Composé A précédemment

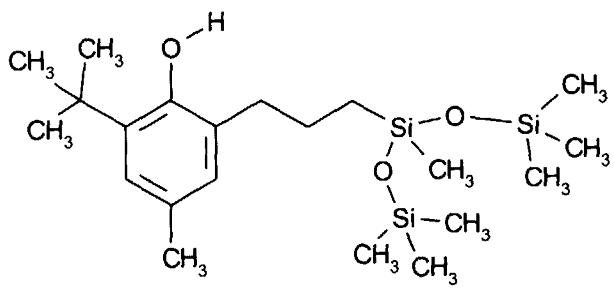

A une solution d'allyl-2-méthyl-4-tert-butyl-6-phénol (9,19 g, 0,045 mole) et de catalyseur (complexe à 3-3.5% poids de Pt dans du cyclovinylméthylsiloxane de Hüls Petrarch PC085 : 10 µl) dans 15 ml de toluène sec porté à 60-70°C, on ajoute goutte à goutte en 30 minutes sous barbotage d'azote et sous agitation 10 g (0,045 mole) d'heptaméthyltrisiloxane. On laisse à cette température pendant 4 heures. On concentre le mélange réactionnel et on obtient après chromatographie sur silice (éluant : Heptane/$CH_2Cl_2$ 95:5) 15,5 g d'une huile jaune pâle du dérivé de l'exemple 1 (Rendement : 80 %):

| - Analyse élémentaire pour $C_{21}H_{42}O_3Si_3$ | | | |
|---|---|---|---|
| théorie : | C59.10 | H9.92 | Si19.74 |
| trouvé : | C59.29 | H9.87 | Si19.47 |

- EXEMPLE 2 :

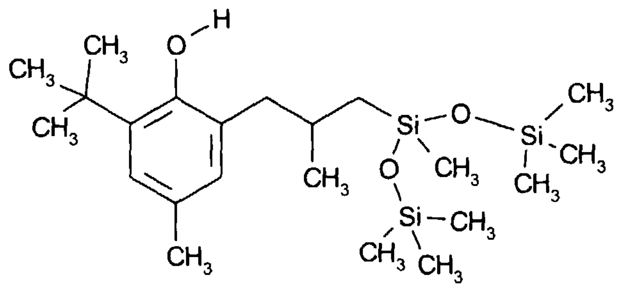

A une solution de methallyl-2-méthyl-4-tert-butyl-6-phénol (15,5 g, 0,071 mole) et de catalyseur (complexe à 3-3.5% poids de Pt dans du cyclovinylméthylsiloxane de Hüls Petrarch PC085 : 100 µl) dans 30 ml de toluène sec porté à 75°C, on ajoute goutte à goutte en 10 minutes sous barbotage d'azote et sous agitation 16,69 g (0,075 mole) d'heptaméthyltrisiloxane. On laisse à cette température pendant 4 heures. On concentre le mélange réactionnel et on obtient après chromatographie sur silice (éluant : Heptane/$CH_2Cl_2$ 95:5) 15 g d'une huile incolore du dérivé de l'exemple 2 (Rendement : 48 %):

| - Analyse élémentaire pour $C_{22}$ $H_{44}$ $O_3$ $Si_3$ | | | |
|---|---|---|---|
| théorie : | C59.94 | H10.06 | Si19.11 |
| trouvé : | C60.21 | H10.08 | Si18.96 |

- EXEMPLE 3 :

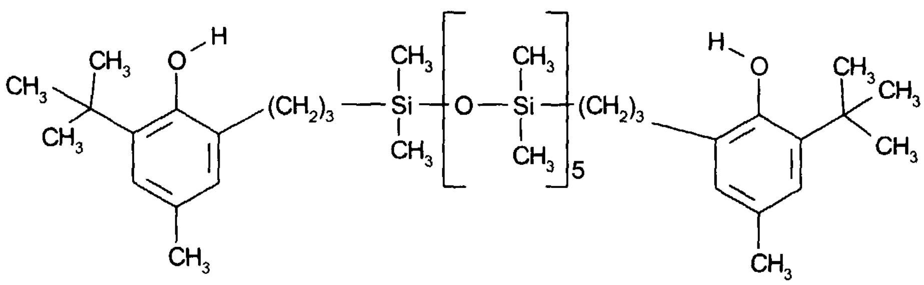

A une solution d'allyl-2-méthyl -4-tert-butyl-6-phénol (9,48 g, 0,046 mole) et de catalyseur (complexe à 3-3.5% poids de Pt dans du cyclovinylméthylsiloxane de Hüls Petrarch PC085 : 20 µl) dans 20 ml de toluène sec porté à 60-70°C, on ajoute goutte à goutte en 30 minutes sous barbotage d'azote et sous agitation 10 g (0,023 mole) de 1,1,3,3,5,5,7,7,9,9,11,11-dodecamethylhexasiloxane. On laisse à 80°C pendant 6 heures. On concentre le mélange réactionnel et on obtient après passage sur lit de silice (éluant : $CH_2Cl_2$) 18 g d'une huile jaune pâle du dérivé de l'exemple 3 (Rendement : 92%) dont les spectres RMN du proton, du carbone et du silicium sont en accord avec la structure.

- EXEMPLE 4 :

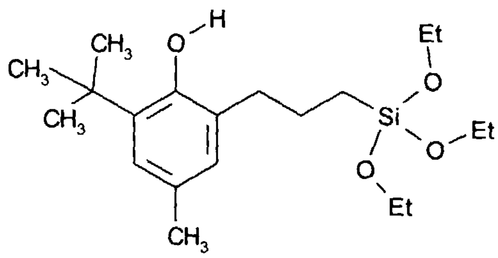

A une solution d'allyl-2-méthyl-4-tert-butyl-6-phénol (50,2 g, 0,246 mole) et de catalyseur (complexe à 3-3.5% poids de Pt dans du cyclovinylméthylsiloxane de Hüls Petrarch PC085 : 200 µl) dans 260 ml de toluène sec porté à 55-60°C, on ajoute goutte à goutte en 30 minutes sous barbotage d'azote et sous agitation 44,8 g (0,273 mole) de triéthoxy silane dissout dans 40 ml de toluène sec. On laisse à cette température pendant 4 heures. On concentre le mélange réactionnel et on obtient après chromatographie sur silice (éluant : Heptane, puis gradiant de $CH_2Cl_2$ dans l'Heptane) 80 g d'une huile jaune pâle du dérivé de l'exemple 4 (Rendement : 84 %):

| - Analyse élémentaire pour $C_{20}$ $H_{36}$ $O_4$ Si | | | |
|---|---|---|---|
| théorie : | C65.17 | H17.36 | Si7.62 |
| trouvé : | C65.28 | H17.52 | Si7.48 |

- EXEMPLE 5 : Appelé Composé B précédemment

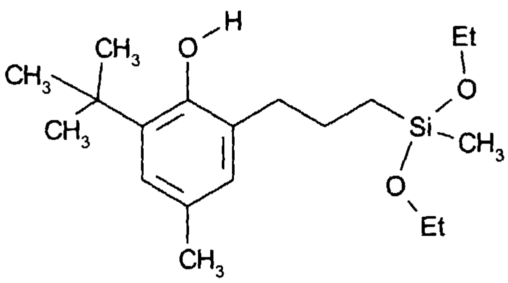

A une solution d'allyl-2-méthyl-4-tert-butyl-6-phénol (20,43 g, 0,1 mole) et de catalyseur (complexe à 3-3.5% poids de Pt dans du cyclovinylméthylsiloxane de Hüls Petrarch PC085 : 50 µl) dans 80 ml de toluène sec porté à 60°C, on ajoute goutte à goutte en 10 minutes sous barbotage d'azote et sous agitation 13,43 g (0,1 mole) de méthyl diéthoxy silane dissout dans 10 ml de toluène sec. On laisse à cette température pendant 3 heures. On concentre le mélange réactionnel et on obtient après chromatographie sur silice (éluant : Heptane puis gradiant de $CH_2Cl_2$ dans l'Heptane) 12 g d'une huile jaune pâle du dérivé de l'exemple 5 (Rendement : 35 %):

| - Analyse élémentaire pour $C_{19}$ $H_{34}$ $O_3$ Si | | | |
|---|---|---|---|
| théorie : | C67.41 | H14.18 | Si8.30 |
| trouvé : | C67.70 | H14.08 | Si8.12 |

- EXEMPLE 6 :

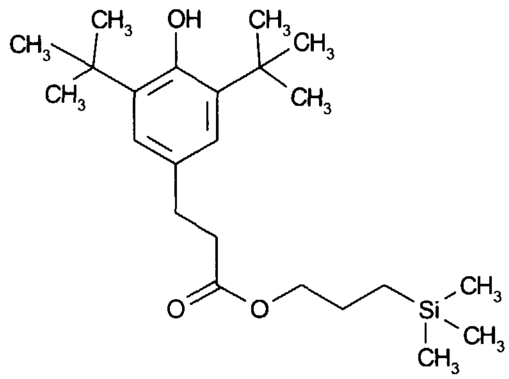

A une solution d'acide 3,5-di-tert-butyl-4-hydroxy-phényl propionique (16,7 g, 0,06 mole) et de carbonate de potassium (9,12 g, 0,06 mole) dans 80 ml de diméthylformamide porté à 80°C, on ajoute goutte à goutte en 30 minutes sous barbotage d'azote et sous agitation 9,95 g (0,06 mole) de 3-chloropropyl triméthylsilane. On laisse à cette température pendant 1 heure 30 minutes. On refroidit et on verse dans l'eau glacée. Le solide obtenu est filtré et recristallisé dans un mélange Eau/Méthanol 10:90 pour obtenir 12 g du dérivé de l'exemple 6 (Rendement : 51 %):

| - Analyse élémentaire pour $C_{23}$ $H_{40}$ $O_3$ Si | | | |
|---|---|---|---|
| théorie : | C70.36 | H10.27 | Si7.15 |
| trouvé : | C70.46 | H10.24 | Si7.02 |

- EXEMPLE 7 :

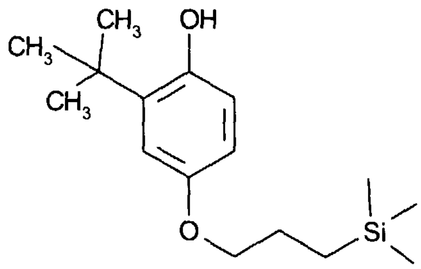

A une solution de 2-tert-butyl-hydroquinone (9,97 g, 0,06 mole) et de carbonate de potassium (9,12 g, 0,066 mole) dans 60 ml de diméthylformamide porté à 80°C, on ajoute goutte à goutte en 30 minutes sous barbotage d'azote et sous agitation 9,95 g (0,06 mole) de 3-chloropropyl triméthylsilane. On laisse à cette température pendant 8 heures. On refroidit et on verse dans l'eau glacée. La phase organique est extraite au dichlorométhane, séchée sur sulfate de sodium puis concentrée. Par chromatographie sur silice (éluant: Heptane/$CH_2Cl_2$ 50:50) on sépare 5,5g d'une huile brune correspondant au dérivé de l'exemple 7 (Rendement : 33):

| - Analyse élémentaire pour $C_{16}$ $H_{28}$ $O_2$ Si | | | |
|---|---|---|---|
| théorie : | C68.52 | H10.06 | Si10.01 |
| trouvé : | C68.22 | H10.03 | Si9.80 |

- EXEMPLE 8 :

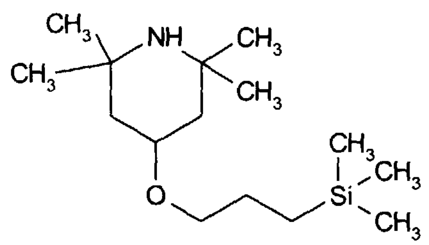

A une solution de 4-hydroxy-2,2,6,6-tetraméthyl piperidine ( 15,7g, 0,1 mole) et de carbonate de potassium (15,2 g, 0,11 mole) dans 50 ml de diméthylformamide porté à 80°C, on ajoute goutte à goutte en 30 minutes sous barbotage d'azote et sous agitation 16,6 g (0,11 mole) de 3-chloropropyl triméthylsilane. On laisse à cette température pendant 24 heures. On refroidit et on verse dans l'eau glacée. La phase organique est extraite au dichlorométhane, séchée sur sulfate de sodium puis concentrée. Par chromatographie sur silice (éluant: $CH_2Cl_2$) on isole 6,3g d'une huile jaune pâle correspondant à l'exemple 8 (Rendement: 23 %) et dont les spectres RMN du proton et du carbone sont en accord avec la formule.

- EXEMPLE 9 : Appelé composé C précédemment

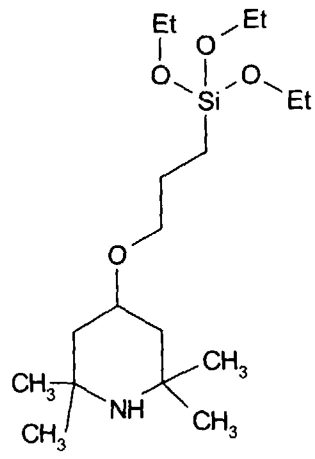

A une solution de 4-allyloxy-2,2,6,6-tetraméthyl piperidine (24 g, 0,122 mole) et de catalyseur (complexe à 3-3.5% poids de Pt dans du cyclovinylméthylsiloxane de Hüls Petrarch PC085 : 100 µl) dans 60 ml de toluène sec porté à 70-75°C, on ajoute goutte à goutte en 30 minutes sous barbotage d'azote et sous agitation 20 g (122 meq en SiH) de triéthoxy silane. On laisse à 70°C pendant 1 heure. On refroidit et on concentre le mélange réactionnel. L'huile obtenue est distillée sous vide de 0,08 mmHg. La fraction distillant à 118-119°C est le dérivé de l'exemple 9 (12,4 g, Rendement : 28 %):

- huile incolore

| - Analyse élémentaire pour $C_{18}$ $H_{39}$ N $O_4$ Si | | | | |
|---|---|---|---|---|
| théorie : | C59.79 | H10.87 | N3,87 | Si7.77 |
| trouvé : | C59.55 | H10.93 | N3,68 | Si7.60 |

- EXEMPLE 10 : Appelé composé E précédemment

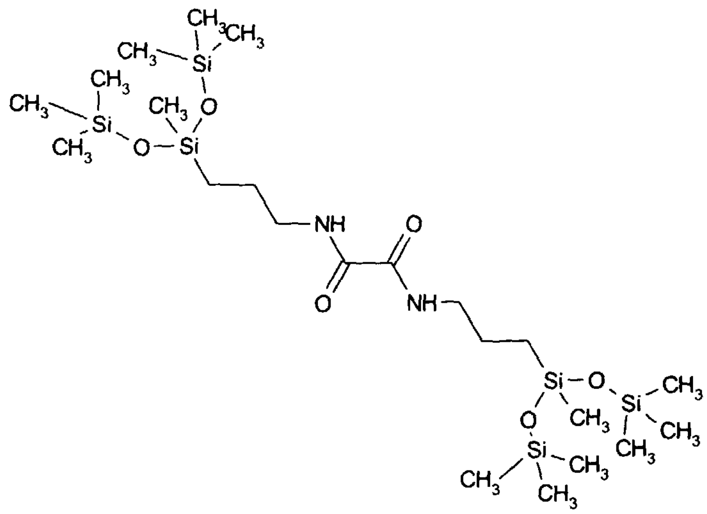

A une solution d'amino-1[1,3,3,3-tetramethyl-1-[(trimethylsilyl) oxy]disiloxanyl]-propane (5,59 g, 0,02 mole) et de triéthylamine (4,04 g, 0,04 mode) dans 20 ml de dimèthylformamide à température du labo, on ajoute par petites portions en 30 minutes sous agitation 1,27 g (0,01 mode) de chlorure d'oxalyle. On laisse sous agitation pendant 6 heures à température du labo. On verse dans l'eau glacé et on extrait au dichloro méthane. La phase organique est lavée à l'eau, séchée et le solvant évaporé pour donner 6,2 g du dérivé de l'exemple 10 (Rendement : 90 %) sous forme d'une huile incolore et dont les spectres RMN du proton et du carbone sont en accord avec la formule.

**EXEMPLES DE FORMULATION**

**EXEMPLE 1 Après-shampooing protecteur de la couleur des cheveux**

| | |
|---|---|
| - Composé A | 2 g |
| - Cyclopentadiméthylsiloxane vendu sous le nom DC245 par DOW CORNING | 15 g |
| - Cyclotétradiméthylsiloxane vendu sous le nom DC244 par DOW CORNING | 15 g |
| - Polydiméthylsiloxane $\alpha,\Omega$-dihydroxyle/silicone volatile vendu sous le nom Q2 1401 par Dow CORNING | 20 g |
| - Ethanol | 5 g |
| - Eau qsp | 100 g |

**EXEMPLE 2 Gel conditionneur après-shampooing protecteur de la couleur des cheveux**

| | |
|---|---|
| - Composé E | 6 g |
| - Polydiméthylsiloxane $\alpha,\Omega$-dihydroxyle/silicone volatile vendu sous le nom Q2 1401 par Dow CORNING | 20 g |

(suite)

| | |
|---|---|
| - Copolymère réticulé acrylamide/acide 2-acrylamido-2-méthylpropane sulfonique vendu sous le nom SEPIGEL 305 par SEPPIC | 1 g MA |
| - Eau qsp | 100 g |

Ce gel permet de réduire sensiblement la dégradation de la couleur des cheveux liée à l'action des agents extérieurs notamment de la lumière.

**<u>EXEMPLE 3</u> Gel conditionneur après-shampooing protecteur de la couleur des cheveux**

| | |
|---|---|
| - Composé B | 6 g |
| - Polydiméthylsiloxane vendu sous le nom SILBIONE V 500000 par RHONE POULENC | 10 g |
| - Copolymère réticulé acrylamide/acide 2-acrylamido-2-méthylpropane sulfonique vendu sous le nom SEPIGEL 305 par SEPPIC | 1 g MA |
| - Eau qsp | 100 g |

Ce gel permet de réduire sensiblement la dégradation de la couleur des cheveux liée à l'action des agents extérieurs notamment de la lumière.

**<u>EXEMPLE 4</u> Shampooing protecteur de la couleur des cheveux**

| | |
|---|---|
| - Composé A | 1 g |
| - Organosiloxane à fonction filtre : composé G | 1 g |
| - Lauryl éther sulfate de sodium à 2,2 moles d'oxyde d'éthylène | 15 g M.A. |
| - Cocoylbétaïne | 2,4 g M.A. |
| - Distéarate d'éthylène glycol | 2,5 g |
| - Monoisopropanolamide d'acide de coprah | 2,5 g |
| - Eau qsp | 100 g |

Ce shampooing présente de bonnes propriétés de lavage et permet après applications et rinçage de réduire sensiblement la dégradation de la couleur des cheveux liée à l'action des agents extérieurs notamment de la lumière.

**<u>EXEMPLES COMPARATIFS</u>**

On effectue une première série de mèches de cheveux naturels châtain foncé.
On effectue sur une seconde série de mèches de cheveux à 90% de blancs légèrement sensibilisés, une teinture par oxydation conduisant à une nuance 《blond foncé doré acajou》.
Les mèches sont ensuite traitées par la composition (1), (2), (3) ou (4) suivante selon le mode opératoire indiquée ci-après :

**COMPOSITION (1) (invention) :**

| | |
|---|---|
| - Composé C | 0,72 % en poids |
| - Ethanol absolu qsp | 100 % en poids |

**COMPOSITION (2) (invention) :**

| | |
|---|---|
| - Composé B | 0,68 % en poids |
| - Ethanol absolu qsp | 100 % en poids |

**COMPOSITION (3) (invention) :**

| | |
|---|---|
| - Composé A | 0,85 % en poids |
| - Ethanol absolu qsp | 100 % en poids |

**COMPOSITION (4) (témoin) :**

| | |
|---|---|
| - Ethanol absolu | 100 % en poids |

***Mode opératoire :***

Chaque mèche est immergée pendant 1 heure à 30°C dans 25 ml de solution. Elle est ensuite essorée entre 2 feuilles de papier (2 fois). Chaque mèche est ensuite rincée avec un mélange eau-éthanol (10-90). Après essorage entre deux doigts, les mèches sont séchées au casque pendant 30 mn à 60 °C.

Les mèches ainsi traitées subissent ensuite un test de résistance à la lumière (Xenotest).

Pour ce faire, les mèches de cheveux ont été fixées sur un support (carton ou plastique). Ces supports ont été disposés sur des porte-échantillons qui tournent autour d'une lampe Xénon pendant une durée de 120 heures sous un taux d'humidité relative de 25 ± 5 % et à une température de 42,5 ± 2,5°C.

Les couleurs des mèches ont été évaluées dans le système L a b, au moyen d'un colorimètre CM 2002 MINOLTA.

On mesure dans ce système pour chaque mèche :

- la couleur de la mèche obtenue après traitement par la composition (1), (2), (3), et (4) et avant le test de résistance à la lumière et
- la couleur de la mèche obtenue après le test de résistance à la lumière.

Selon ce système, L indique la clarté. Plus la valeur de L est élevée, plus la nuance est claire. Inversement, Plus la valeur de L est faible, plus la nuance est sombre.

La nuance et la saturation sont exprimées par a et b.

a et b indiquent deux axes de nuances, a indiquant l'axe des rouges/verts et b l'axe des jaunes/bleus. Les valeurs proches de zéro pour a ou b correspondent aux nuances grises.

La différence de couleur de chaque mèche avant et après le test de résistance à la lumière reflète la dégradation de la coloration due à l'action de la lumière et a été calculée en appliquant l'équation suivante :

$$\Delta E = \sqrt{(L-L_o)^2+(a-a_o)^2+(b-b_o)^2}$$

Dans cette formule, Δ**E** représente la différence de couleur entre deux mèches, L, a et b représentent respectivement la clarté, la nuance et la saturation après le test et $L_0$, $a_0$ et $b_0$ représentent respectivement la clarté, la nuance et la saturation avant le test.

Les résultats des tests sont indiqués dans les tableau I et II ci-dessous :

Tableau I

| **Composition** | **Couleur avant le test sur cheveux naturels châtain** | | | **Couleur après le test sur cheveux naturels châtain** | | | **Dégradation de la couleur ΔE. sur cheveux naturels châtain** |
|---|---|---|---|---|---|---|---|
| | **L** | **a** | **b** | **L** | **a** | **b** | |
| **(1) (invention)** | 22,1 | 2,2 | 2,8 | 27,6 | 4,1 | 8,1 | 7,9 |
| **(2) (invention)** | 21,7 | 2,1 | 2,9 | 28,5 | 3,9 | 8,0 | 8,6 |
| **(4) (témoin)** | 21,7 | 2,2 | 2,8 | 30,8 | 4,8 | 10,6 | 12,2 |

On constate que les compositions (1) et (2) selon l'invention contenant une silicone antioxydante réduisent respectivement de 35 et de 29 % la dégradation de la couleur des mèches, après 120 heures d'exposition à la lumière, par rapport à la composition (4) dépourvue de silicone antioxydante.

Tableau II

| **Composition** | **Couleur avant le test sur cheveux teints en blond foncé doré acajou** | | | **Couleur après le test sur cheveux teints en blond foncé doré acajou** | | | **Dégradation de la couleur ΔE. sur cheveux teints en blond foncé doré acajou** |
|---|---|---|---|---|---|---|---|
| | **L** | **a** | **b** | **L** | **a** | **b** | |
| **(2) (invention)** | 27,4 | 2,5 | 6,2 | 53,7 | 2,2 | 14,5 | 27,6 |
| **(3) (invention)** | 27,2 | 2,4 | 5,7 | 53,6 | 2,3 | 15,2 | 28,0 |
| **(4) (témoin)** | 25,6 | 2,5 | 5,1 | 56,1 | 2,4 | 17,0 | 32,7 |

On constate que les compositions (2) et (3) selon l'invention contenant une silicone antioxydante réduisent respectivement de 16 et de 14 % la dégradation de la couleur des mèches teintes artificiellement, après 120 heures d'exposition à la lumière, par rapport à la composition (4) dépourvue de silicone antioxydante.

## Revendications

**1.** Utilisation d'au moins un organosiloxane et/ou d'au moins un organosilane porteur d'au moins un groupe antioxydant pour la préparation d'une composition cosmétique ou dermatologique comme agent protecteur de la couleur des fibres kératiniques colorées naturellement ou teintes artificiellement.

**2.** Utilisation selon la revendication 1, caractérisée par le fait que l'organosiloxane et/ou l'organosilane comporte dans sa molécule au moins une unité de formule (I) suivante :

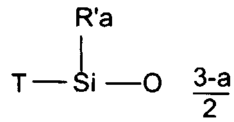 (I)

dans laquelle :

$R'_a$, identiques ou différents, désignent un groupe hydrocarboné saturé ou insaturé en $C_1$-$C_{30}$, un groupe hydrocarboné halogéné en $C_1$-$C_8$, un groupe hydrocarboné aromatique, un groupe alkoxy en $C_1$-$C_{10}$,
a = 1, 2 ou 3 ;
T = -E-U où E représente un radical divalent hydrocarboné aliphatique, saturé ou insaturé, linéaire ou ramifié ayant au moins 2 atomes de carbone et renfermant éventuellement un ou plusieurs atomes d'oxygène ou bien un groupement carboxylique, amide ou urée ou bien un groupe aromatique ou bien un radical hydroxyle et U représente le reste d'une molécule antioxydante.

**3.** Utilisation selon la revendication 2, caractérisée par le fait que l'organosiloxane comporte en plus des unités de formule (I), des unités de formule (II) et/ou des unités de formule (III) :

$$R'b - Si - O_{\frac{4-b}{2}} \quad (II)$$

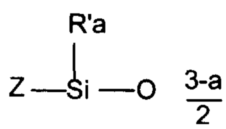 (III)

dans lesquelles :

R' et a ont les même significations que dans la formule (I) ;
b = 1, 2 ou 3 ;
Z = -OU, U ayant la même signification que dans la formule (I).

**4.** Utilisation selon l'une quelconque des revendications 2 à 3, où dans la molécule de l'organosiloxane constituée d'unités de formule (I) et éventuellement d'unités de formule (II) et/ou de formule (III), au moins 40% en nombre des radicaux R' sont des radicaux méthyle et le nombre total des unités (I), (II) et (III) est inférieur ou égal à 250 et varie en particulier de 2 à 50.
**5.** Utilisation selon l'une quelconque des revendications 2 à 4, selon laquelle, dans la molécule de l'organosiloxane constituée d'unités de formule (I) et éventuellement d'unités de formule (II) et/ou de formule (III), U représente un reste choisi parmi : U représente un reste choisi dans le groupe constitué par :

- un radical phénol mono ou di-ortho substitué et dont le noyau benzénique peut être substitué par un ou plusieurs radicaux alkyle, halogène, hydroxy, alkoxy, alkylcarbonyle;
- un dérivé 1,1,6,6-tetrasubstitué de piperidine (HALS);
- un groupe oxanilide substitué par un ou plusieurs radicaux alkyle, halogène, hydroxy, alkoxy
- un groupe oxamide.

**6.** Utilisation selon l'une quelconque des revendications 1 à 5, selon laquelle l'organosiloxane porteur d'au moins un groupe antioxydant est choisi parmi ceux à fonction ter-butyl phénol et/ou dérivé 1,1,6,6-tetrasubstitué de piperidine et/ou dérivé d'oxamide et/ou dérivé d'oxanilide qui répondent aux formules suivantes:

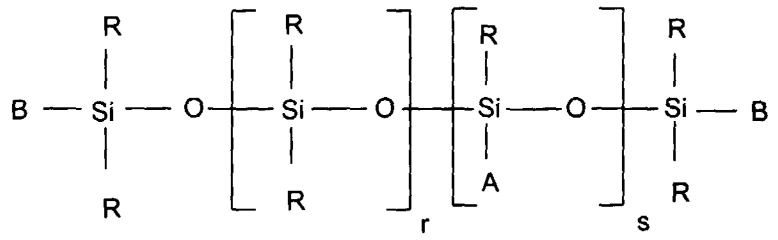

(1)

ou

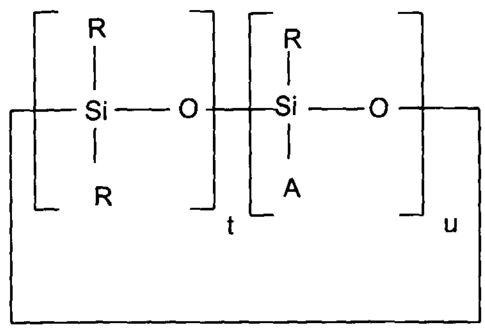

(2)

ou l'organosilane porteur d'au moins un groupe antioxydant est choisi parmi ceux à fonction ter-butyl phénol et/ou dérivé 1,1,6,6-tetrasubstitué de piperidine et/ou dérivé d'oxamide et/ou dérivé d'oxanilide qui répond à la formule suivante:

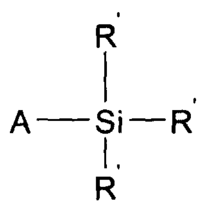

(3)

formules (1), (2) et (3) dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- R', identiques ou différents sont choisis parmi les radicaux alkyles en $C_1$-$C_{10}$, linéaires ou ramifiés ou les radicaux alkoxy en $C_1$-$C_8$,
- et le symbole A désigne un radical monovalent lié directement à un atome de silicium, et qui répond aux formules (4a), (4b) et (4c) suivantes :

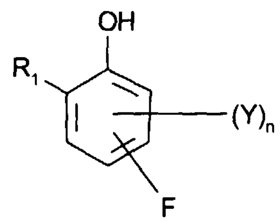

(4a)

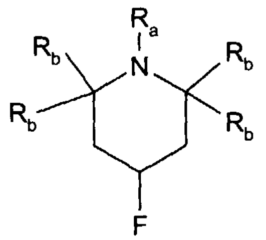

(4b)

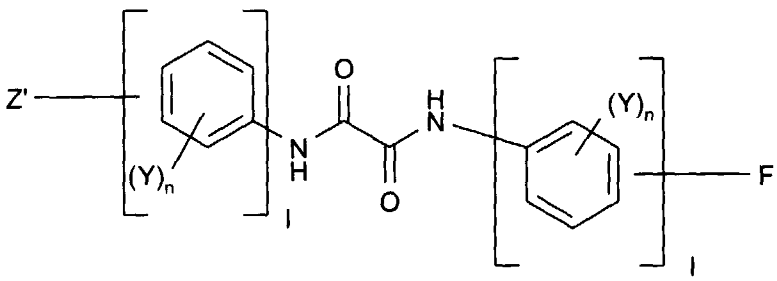

(4c)

formules (4) dans lesquelles :

F est le biradical de formule (5) suivante :

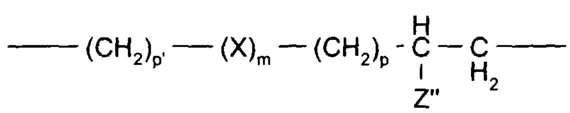

(5)

- $R_1$ est un radical alkyle en $C_3$-$C_{12}$ ramifié ou un radical alkoxy en $C_1$- $C_4$,
- Y, identiques ou différents, sont choisis parmi les radicaux alkyles en $C_1$-$C_{12}$, linéaires ou ramifiés, les radicaux cycloalkyles en $C_5$-$C_7$, le radical hydroxyle, les radicaux alkyle alkoxy, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- n est un nombre entier compris entre 0 et 2 inclusivement,
- X représente O ou $NR_c$ ou O(C=O) ou $NR_c$(C=O) ou $NR_c$(C=O)O avec $R_c$ qui représente l'hydrogène ou un radical alkyle en $C_1$-$C_4$,
- Z" représente l'hydrogène ou un radical alkyle en $C_1$-$C_4$,
- m est 0 ou 1,
- p et p' représentent un nombre entier compris entre 0 et 10, inclusivement.
- $R_a$ représente l'hydrogène, l'oxygène (radical nitroxyde), un radical alkyle en $C_1$-$C_8$, un radical hydroxyle, un radical hydroxyalkyle en $C_2$-$C_4$, un radical alkylcarbonyle, phényle, benzyle ou un radical alkoxy en $C_1$-$C_6$ linéaire ou cyclique
- $R_b$ représente un radical alkyle en $C_1$-$C_8$

- l est 0 ou 1,
- Z' représente le radical divalent F, l'hydrogène ou une chaîne hydrocarbonée, linéaire ou ramifié, saturée ou insaturée comportant jusqu'à 20 atomes de carbone consécutifs, pouvant comporter un ou plusieurs groupements hydroxyle et pouvant être interrompue par un ou plusieurs atomes d'oxygène ou d'azote et/ou plusieurs cycles aromatiques.

**7.** Utilisation selon la revendication 6, caractérisée par le fait que l'organosiloxane à fonction antioxydante est choisi parmi les composés de formule générale (1) pour lesquels les radicaux B sont choisis parmi les radicaux R.
**8.** Utilisation selon la revendication 6 ou 7, caractérisée par le fait que l'organosiloxane à fonction antioxydante est choisi parmi les composés de formule générale (1) présentant au moins l'une des caractéristiques suivantes :

- B est un radical R,
- R est alkyle et encore plus préférentiellement est méthyle,
- r est compris entre 0 et 15 inclusivement ; s est compris entre 1 et 10 inclusivement,
- n est non nul, et de préférence égal à 1 , et Y est alors choisi parmi méthyle, ter.-butyle ou alcoxy en $C_1$-$C_4$,
- Z" est hydrogène ou méthyle,
- m=0, ou [m=1 et X=O]
- p est égal à 1.

**9.** Utilisation selon la revendication 8, caractérisée par le fait que l'organosiloxane à fonction antioxydante présente l'ensemble desdites caractéristiques.
**10.** Utilisation selon l'une quelconque des revendications 6 à 9, caractérisée par le fait que l'organosiloxane à fonction antioxydante correspond à la formule générale (6) suivante :

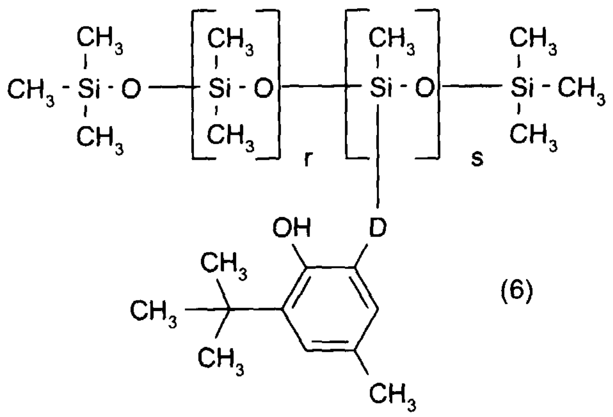

(6)

avec

$$0 \leq r \leq 6,$$

$$1 \leq s \leq 6,$$

et où D représente le radical divalent :

$$—CH_2—CH_2—CH_2—$$

**11.** Utilisation selon la revendication 10, caractérisée par le fait que l'organosiloxane à fonction antioxydante correspond à la formule :

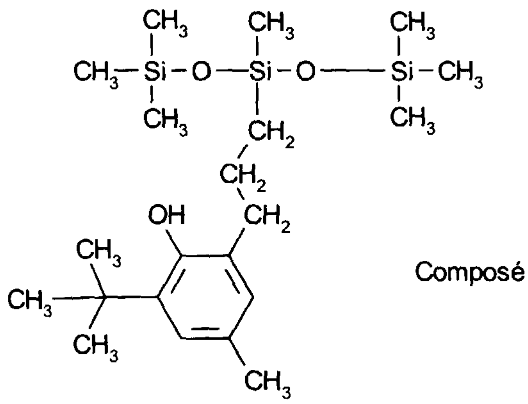

Composé A

**12.** Utilisation selon l'une quelconque des revendications 6 à 9, caractérisée par le fait que l'organosiloxane à fonction antioxydante correspond à la formule suivante (7) :

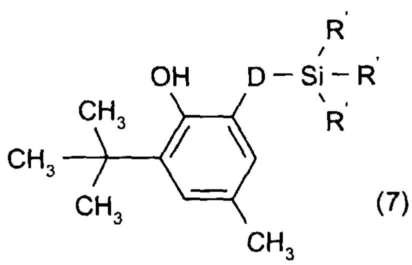

(7)

ou R' a la même définition qu'au dessus

et où D représente le radical divalent :

$$-CH_2-CH_2-CH_2-$$

**13.** Utilisation selon la revendication 12, caractérisée par le fait que l'organosiloxane à fonction antioxydante correspond à la formule :

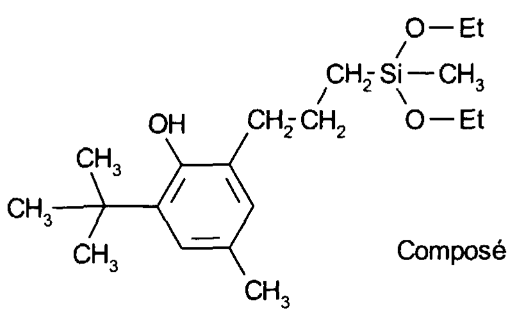

Composé B

**14.** Utilisation selon l'une quelconque des revendications 6 à 9, caractérisée par le fait que l'organosiloxane à fonction antioxydante correspond à la formule suivante (8) :

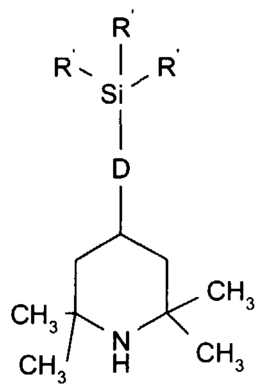

(8)

ou R' a la même définition qu'au dessus et où D représente le radical divalent :

$$—O—CH_2—CH_2—CH_2—$$

**15.** Utilisation selon la revendication 14, caractérisée par le fait que l'organosiloxane à fonction antioxydante correspond à la formule :

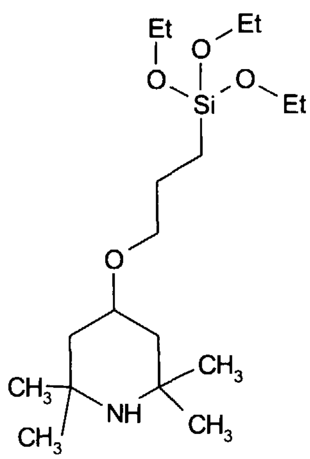

Composé C

**16.** Utilisation selon l'une quelconque des revendications 6 à 9, caractérisée par le fait que l'organosiloxane à fonction antioxydante correspond à la formule suivante (9) :

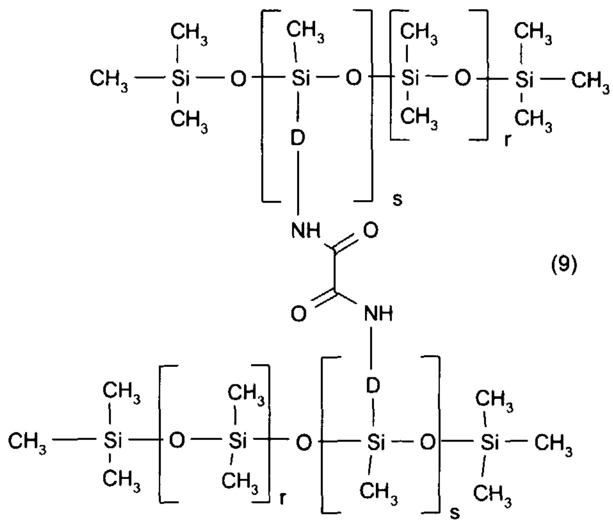

(9)

avec

$$0 \leq r \leq 6,$$

$$1 \leq s \leq 6,$$

et où D représente le radical divalent :

$$-CH_2-CH_2-CH_2-$$

**17.** Utilisation selon la revendication 16, caractérisée par le fait que l'organosiloxane à fonction antioxydante correspond à la formule :

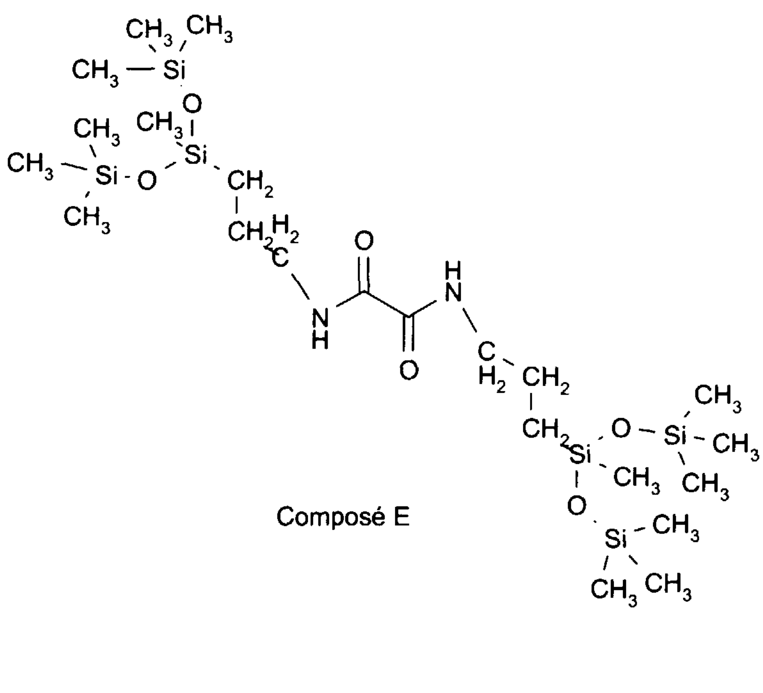

Composé E

**18.** Utilisation selon l'une quelconque des revendications 1 à 17, où l'organosiloxane à groupement antioxydant est associé à moins un organosiloxane porteur d'au moins un groupe absorbant les ultraviolets.
**19.** Utilisation selon la revendication 18, où l'organosiloxane porteur d'au moins un groupe absorbant les ultraviolets répond à la formule suivante :

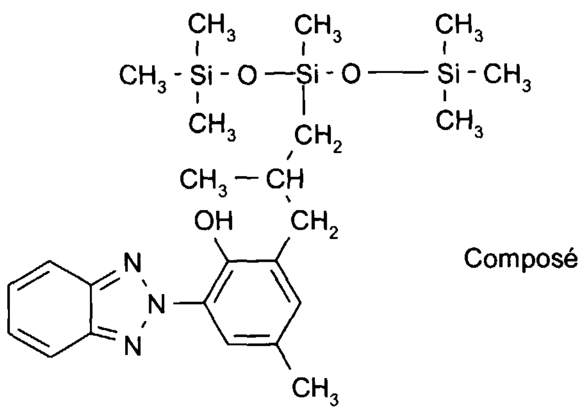

Composé G

**20.** Composition photoprotectrice de la couleur naturelle ou artificielle des fibres kératiniques, en particulier des cheveux humains, comprenant dans un support cosmétiquement acceptable au moins un organosiloxane et/ou un organosilane à fonction antioxydante tel que défini selon l'une quelconque des revendications 1 à 18 à et au moins un organosiloxane porteur d'au moins un groupe absorbant les ultraviolets.

**21.** Composition selon la revendication 20, où l'organosiloxane porteur d'au moins un groupe absorbant les ultraviolets répond à la formule suivante :

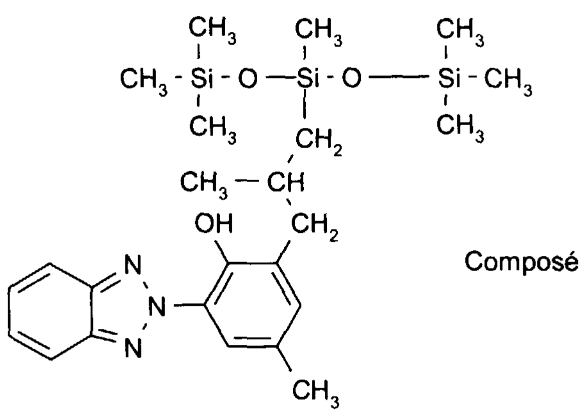

Composé G

**22.** Utilisation selon l'une quelconque des revendications 1 à 21, selon laquelle l'organosiloxane portant un groupe antioxydant est utilisé dans des quantités au moins égales à 0,01% en poids et généralement allant de 0,1 à 20% en poids et plus particulièrement de 1 à 10% en poids par rapport au poids de la composition destinée à être appliquée sur les fibres kératiniques colorées naturellement ou teintes artificiellement.

**23.** Utilisation selon l'une quelconque des revendications 1 à 22, selon laquelle la composition destinée à être appliquée sur les fibres kératiniques se présente sous forme de lotion huileuse, de lotion alcoolique, de lotion hydroalcoolique, d'émulsion, de dispersion aqueuse ou hydroalcoolique.

**24.** Utilisation selon l'une quelconque des revendications 1 à 22, pour la préparation d'un produit capillaire à rincer après application.

**25.** Utilisation selon l'une quelconque des revendications 1 à 22, pour la préparation d'un produit capillaire non rincé après application

**26.** Utilisation selon l'une quelconque des revendications 1 à 22, pour la préparation d'un produit capillaire appliqué avant, pendant ou après teinture des cheveux.

**27.** Organosilane répondant à l'une des formules (10a) et (10b) suivantes :

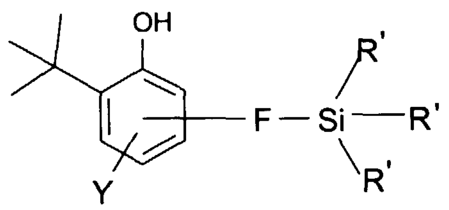

(10a)

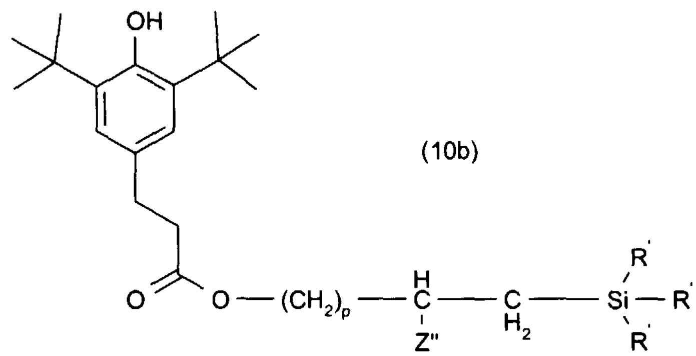

(10b)

dans lesquelles F, p, Z", Y et R' ont les significations indiquées dans l'une quelconque des revendications 6 à 9; sous réserve que R' soit différent d'un radical alcoxy.

**28.** Organosilane rép

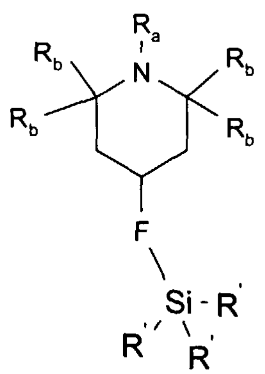

(11)

ondant à la formule (11) suivante : dans laquelle $R_a$, $R_b$, F et R' ont les significations indiquées dans l'une quelconque des revendications 6 à 9 ; sous réserve que R' soit différent d'un radical alcoxy.

**30.** Organosiloxane répondant à l'une des formules (1) ou (2) telle que définie dans la revendication 6 dans lesquelles A répond à la formule :

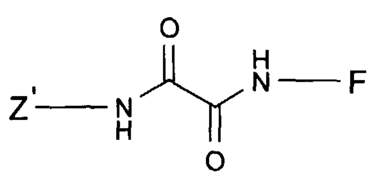

où F et Z' ont les significations indiquées dans l'une quelconque des revendications 6 à 9.

**31.** Organosilane répondant à la formule (12) suivante :

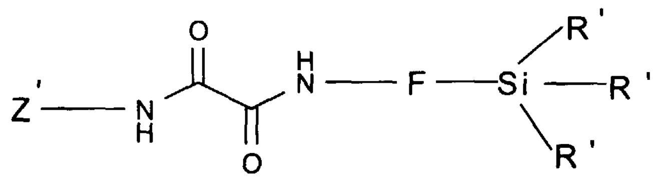

(12)

où F, Z' et R' ont les significations indiquées dans l'une quelconque des revendications 6 à 9 ; sous réserve que R' soit différent d'un radical alcoxy.

**32.** Utilisation d'au moins un organosiloxane et/ou d'au moins un organosilane porteur d'au moins un groupe antioxydant tel que défini dans l'une quelconque des revendications 27 à 31 pour la préparation d'une composition cosmétique ou dermatologique comme agent protecteur de la couleur des fibres kératiniques colorées naturellement ou teintes artificiellement.

**33.** Composition photoprotectrice de la couleur naturelle ou artificielle des fibres kératiniques, en particulier des cheveux humains, comprenant dans un support cosmétiquement acceptable au moins un organosiloxane et/ou au moins un organosilane à fonction antioxydante tel que défini selon l'une quelconque des revendications 27 à 31.

Office européen des brevets

# RAPPORT DE RECHERCHE EUROPEENNE

Numéro de la demande
EP 98 40 3312

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP 0 509 922 A (L'OREAL) 21 octobre 1992<br>* le document en entier *<br>--- | 1-26,32, 33 | C07F7/08<br>C07F7/18<br>C07F7/21<br>C08G77/388<br>A61K7/06 |
| X | CHEMICAL ABSTRACTS, vol. 126, no. 9, 3 mars 1997<br>Columbus, Ohio, US;<br>abstract no. 119883,<br>HIRAI, MOTOHIKO: "Silicone mold-releasing lubricating oil compositions containing antioxidants"<br>XP002083927<br>* abrégé *<br>& JP 08 311348 A (SHINETSU CHEM IND CO, JAPAN)<br>--- | 27 | |
| X | CHEMICAL ABSTRACTS, vol. 107, no. 4, 27 juillet 1987<br>Columbus, Ohio, US;<br>abstract no. 23817,<br>KIYOTSUKURI, TSUYOSHI ET AL: "Preparation and properties of silicon-containing polyamides"<br>XP002083928<br>* abrégé *<br>& J. POLYM. SCI., PART A: POLYM. CHEM. (1987), 25(6), 1591-602 CODEN: JPACEC, 1987,<br>--- | 31 | DOMAINES TECHNIQUES RECHERCHES (Int.Cl.6)<br>C07F<br>C08G<br>A61K |
| X | EP 0 162 523 A (ENICHEM SINTESI SPA) 27 novembre 1985<br>* le document en entier *<br>--- | 27 | |
| X | EP 0 491 659 A (CIBA-GEIGY SPA) 24 juin 1992<br>* les exemples *<br>---<br>-/-- | 28 | |

Le présent rapport a été établi pour toutes les revendications

| Lieu de la recherche | Date d'achèvement de la recherche | Examinateur |
|---|---|---|
| LA HAYE | 7 mai 1999 | Rinkel, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 98 40 3312

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| P,X | DATABASE WPI<br>Section Ch, Week 9836<br>Derwent Publications Ltd., London, GB;<br>Class D21, AN 98-414594<br>XP002102218<br>& AU 48341 97 A (UV ECLIPSE PTY LTD)<br>, 25 juin 1998<br>* abrégé *<br>----- | 1-26,32,<br>33 | |
| | | | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 7 mai 1999 | Rinkel, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO. EP 98 40 3312

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

07-05-1999

| Document brevet cité au rapport de recherche | Date de publication | Membre(s) de la famille de brevet(s) | Date de publication |
|---|---|---|---|
| EP 509922 A | 21-10-1992 | FR 2675378 A | 23-10-1992 |
| | | AT 139688 T | 15-07-1996 |
| | | CA 2066363 A | 20-10-1992 |
| | | DE 69211754 D | 01-08-1996 |
| | | ES 2089437 T | 01-10-1996 |
| | | JP 5124938 A | 21-05-1993 |
| | | US 5609856 A | 11-03-1997 |
| EP 162523 A | 27-11-1985 | AT 62263 T | 15-04-1991 |
| | | BR 8502494 A | 28-01-1986 |
| | | CA 1291585 A | 29-10-1991 |
| | | DK 223085 A | 22-11-1985 |
| | | JP 1935367 C | 26-05-1995 |
| | | JP 6060309 B | 10-08-1994 |
| | | JP 61043692 A | 03-03-1986 |
| | | US 4888375 A | 19-12-1989 |
| EP 491659 A | 24-06-1992 | IT 1243409 B | 10-06-1994 |
| | | CA 2057581 A | 18-06-1992 |
| | | CS 9103834 A | 15-07-1992 |
| | | DE 69124352 D | 06-03-1997 |
| | | DE 69124352 T | 09-10-1997 |
| | | JP 4295488 A | 20-10-1992 |
| | | SK 278485 B | 09-07-1997 |
| | | US 5321066 A | 14-06-1994 |
| | | US 5418267 A | 23-05-1995 |
| | | US 5578665 A | 26-11-1996 |
| | | US 5219905 A | 15-06-1993 |

EPO FORM P0460

Pour tout renseignement concernant cette annexe : voir Journal Officiel de l'Office européen des brevets, No.12/82